# FASCICULE DE BREVET EUROPEEN

(11) **EP 2 136 752 B1**
(45) Date de publication et mention de la délivrance du brevet: **29.09.2010**
(21) Numéro de dépôt: 08788169.4
(22) Date de dépôt: 11.04.2008
(51) Int. Cl.: A61F 13/00

(54) **NOUVEAU PROTECTEUR POUR PANSEMENTS**
NEUARTIGER BANDAGENSCHUTZ
NOVEL BANDAGE PROTECTION

(30) Priorité: 13.04.2007 FR 0754452
(43) Date de publication de la demande: 30.12.2009
(73) Titulaire: Laboratoires Urgo, 21300 Chenove (FR)
(72) Inventeur: QUINON, Etienne, F-21370 Plombieres Les Dijon (FR)
(74) Mandataire: Hubert, Philippe
(86) Numéro de dépôt international: PCT/FR2008/050650
(87) Numéro de publication internationale: WO 2008/145884

(56) Documents cités:
- EP-A- 0 065 399
- WO-A-00/54755
- WO-A-02/02042
- FR-A- 1 326 264
- FR-A- 2 870 113
- US-A- 4 182 449

## Description

La présente invention concerne un nouvel ensemble pansement-protecteur spécifiquement conçu pour permettre le conditionnement, la conservation et la manipulation de pansements incorporant un corps gras, notamment au sein d'une masse susceptible de fluer. Elle concerne également un procédé de fabrication de ce nouvel ensemble pansement-protecteur. Des pansements sont connus de EP-A-0 065399, WO 00/54755 et FR-A-1 326 264.

Dans la présente description, on entend désigner par «corps gras » toute substance ou tout mélange de substances choisie(s) parmi les huiles, les graisses et les corps lipidiques (comprenant les acides gras, glycérols, stérols et leurs dérivés) d'origine naturelle (minérale, animale ou végétale) ou synthétique et se présentant à l'état liquide, semi-solide ou solide, ces substances pouvant être de poids moléculaire et d'architecture (monomérique ou polymérique) variées.

Les protecteurs sont couramment utilisés dans le domaine des pansements. Leur rôle est de protéger la surface du pansement qui est destinée à venir en contact avec la peau ou la plaie du patient , jusqu'au moment où le pansement est appliqué. Généralement, les protecteurs ne couvrent que la surface du pansement qui est destinée à venir en contact avec la peau ou la plaie et se présentent sous la forme d'une feuille de dimension au moins égale à celle de la surface à recouvrir. Les protecteurs peuvent être en une ou plusieurs (habituellement deux ou trois) parties et présenter des pliages formant des ailettes de préhension facilitant leur retrait. Les protecteurs sont, en général, réalisés dans des matériaux anti-adhérents afin de faciliter leur retrait lors de l'application du pansement.

Les pansements incorporant un corps gras, notamment au sein d'une masse, adhésive ou non, sont généralement difficiles à conditionner, à conserver et à manipuler dans les meilleures conditions d'hygiène, en raison des problèmes de migration du corps gras ou de fluage de la masse.

La présente invention a pour but de pallier les inconvénients de ces pansements connus en proposant un ensemble pansement-protecteur d'une nouvelle conception, qui permette d'éviter les problèmes de migration du corps gras ou de fluage de la masse lors du conditionnement et de la conservation du pansement, ainsi que les problèmes d'hygiène lors de sa mise en place sur la peau ou sur la plaie du patient à traiter.

Ainsi, selon un premier aspect, la présente invention a pour objet un ensemble pansement-protecteur, du type comprenant :
- un pansement incorporant au moins un corps gras et présentant une première face destinée à venir au contact de la peau ou de la plaie et une deuxième face opposée à la première ;
- un protecteur séparable dudit pansement,
**caractérisé en ce que** ledit protecteur est constitué d'une feuille unique recouvrant complètement ladite deuxième face et présentant des ailes rabattues sur ladite première face, de façon à la recouvrir sensiblement entièrement.

L'originalité de l'ensemble pansement-protecteur selon l'invention réside dans la structure particulière du protecteur qui est constitué en une seule pièce et qui est destiné à entourer complètement le pansement. Ce protecteur permet d'éviter d'une façon particulièrement simple les problèmes de migration du corps gras ou de fluage de la masse tout en garantissant une mise en place du pansement sur la peau ou sur la plaie dans les meilleures conditions d'hygiène. Il permet en outre, selon le type de pansement utilisé, d'en faciliter la pose et peut dans certains cas jouer également le rôle de support.

D'une façon générale, les ailes rabattues de la feuille précitée formant le protecteur recouvrent entièrement la face du pansement destinée à venir au contact de la peau ou de la plaie, éventuellement à l'exception de la zone de surface extrêmement limitée se situant entre les bords libres de la feuille lorsque ceux-ci sont conçus pour venir en aboutement, en position d'utilisation.

Avantageusement, les ailes rabattues de la feuille précitée formant le protecteur se recouvrent partiellement au niveau de leurs bords libres (latéraux), de préférence de telle façon que la surface de recouvrement ainsi définie représente 10 à 30 %, de préférence encore 10 à 20 % de la surface de la face du pansement recouverte par lesdites ailes.

La surface définie par les ailes précitées est donc au moins égale à celle de la face du pansement qu'elles recouvrent. Ces ailes peuvent éventuellement s'étendre latéralement au-delà des bords longitudinaux du pansement en formant ainsi des organes de préhension qui sont notamment utiles lorsque les bords libres de la feuille sont conçus pour venir en aboutement, en position d'utilisation.

Selon une caractéristique particulière, la feuille formant le protecteur comprend au moins une amorce de pliage, de préférence linéaire, destinée à être positionnée au niveau d'un bord latéral du pansement et permettant son repli sur elle-même pour former l'une des ailes précitées.

Alternativement, la feuille formant le protecteur peut comprendre au moins deux amorces de pliage, de préférence linéaires, destinées à être positionnées respectivement au niveau de chacun des bords latéraux du pansement et permettant son repli sur elle-même pour former respectivement les deux ailes précitées.

Pour faciliter le retrait du protecteur, la feuille le constituant peut-être :
- soit repliée sur elle-même au niveau de l'un de ses bords libres en formant ainsi une ailette de préhension ;
- soit repliée sur elle-même au niveau de chacun de ses bords libres en formant ainsi deux ailettes de préhension.

Les différents types de pliages connus peuvent être envisagés pour former ces ailettes de préhension.

La feuille formant le protecteur peut être en tout matériau ayant une mémoire et à travers laquelle le corps gras contenu dans le pansement ne migre pas.

Par exemple, cette feuille peut être en papier siliconé, polypropylène, polyamide, polyéthylène, polyuréthane, polyester, à base de copolymère de polyéther polyester (comme par exemple les produits commercialisés par la société DuPont sous la dénomination Hytrel^{®}), à base de copolymères de polyester ou polyéther polyuréthanes (comme par exemple les produits commercialisés par la société Noveon sous la dénomination Estane^{®}), à base de copolymères polyéther polyamide (comme par exemple les produits commercialisés par la société Arkema sous la dénomination Pebax^{®}), en polyéther, polychlorure de vinyle, polychlorure de vinylidène, en alcool polyvinylique, en polyacétate de vinyle, en polystyrène, en polyoléfine comme par exemple en polyéthylène ou en polypropylène, en fluorure polyvinylique.

Selon une caractéristique particulière, le matériau constituant la feuille précitée est choisi parmi un papier siliconé, le polyéthylène ou un polyester.

Selon une autre caractéristique particulière, cette feuille présente une épaisseur comprise entre 20 et 200 µm, de préférence entre 40 et 80 µm.

De manière préférentielle, la feuille utilisée pour former le protecteur selon la présente invention est une feuille de polyester non siliconé ayant une épaisseur de 50 µm.

Selon encore une autre caractéristique particulière, cette feuille peut-être recouverte au moins en partie sur une de ses faces par un matériau anti-adhérent ou par un matériau adhésif.

Avantageusement, la feuille précitée constituant le protecteur peut présenter, au niveau de chacune des amorces de pliage, des moyens de pré-découpe. Dans ce cas, les parties formant ailes du protecteur peuvent être retirées par découpe tandis que la partie du protecteur recouvrant la face opposée à la face destinée à venir au contact de la peau ou de la plaie est maintenue en place en servant alors de support pour le pansement.

Selon un premier mode de réalisation, l'ensemble pansement-protecteur selon l'invention comprend un pansement interface.

De tels pansements sont connus et commercialisés par exemple sous les dénominations commerciales Tulle Gras^{®} (par SOLVAY PHARMA), Physiotulle^{®} (par COLOPLAST) ou encore Urgotul^{®} (par les Laboratoires URGO).

Ces pansements interfaces se présentent généralement sous forme d'une trame ou d'un filet enduit d'une masse comprenant au moins un corps gras. Ils peuvent également être constitués d'une masse comprenant au moins un corps gras sans trame ou filet, ayant la forme d'une plaque présentant ou non des trous traversants, en fonction du type de plaie sur lequel le pansement est appliqué (on utilisera préférentiellement une plaque présentant des trous traversants sur une plaie exsudative lorsque la masse comprenant au moins un corps gras n'a qu'un faible ou aucun pouvoir absorbant, les trous permettant ainsi l'évacuation des exsudats de la plaie).

Ces pansements interfaces sont généralement conditionnés dans des pochettes individuelles en y étant éventuellement maintenus entre deux feuilles protectrices pour éviter leur contact direct avec les parois de la pochette de conditionnement.

Ces feuilles protectrices sont généralement faites d'un matériau au travers duquel le corps gras ne migre pas (par exemple en cellophane dans le cas du Tulle Gras^{®} ou en polyester dans le cas d' Urgotul^{®}). Elles permettent donc de protéger la pochette de conditionnement contre toute migration de corps gras qui pourrait entraîner sa détérioration.

Ces feuilles protectrices assurent également une protection supplémentaire du pansement lors de son utilisation. Après avoir ouvert la pochette de conditionnement, l'utilisateur peut en effet retirer le pansement de son conditionnement sans avoir à le toucher directement, en le gardant maintenu entre ces deux feuilles protectrices jusqu'à son utilisation finale.

Toutefois, lorsque le pansement interface est ainsi protégé, le retrait des feuilles protectrices doit se faire en deux temps (en commençant habituellement par le retrait de la feuille protégeant la surface destinée à venir au contact de la peau lésée ou de la plaie) car ces feuilles sont indépendantes l'une de l'autre. De ce fait, lorsque la surface du pansement destinée à être mise au contact de la plaie ou de la peau lésée est libérée de sa feuille protectrice, il n'y a souvent pas d'autre solution que de saisir le pansement à la main pour le tendre sur la peau, avec les risques corrélatifs de polluer le pansement ou de retirer une partie de la masse comprenant au moins un corps gras.

L'ensemble pansement-protecteur selon la présente invention permet non seulement d'éviter les problèmes de migration du corps gras, mais garantit en outre une pose aisée et parfaitement hygiénique du pansement.

En effet, lors de l'utilisation de cet ensemble, le protecteur est séparé du pansement en éloignant ses bords libres l'un de l'autre ce qui permet ainsi de libérer, dans un premier temps, la face du pansement destinée à être mise en contact avec la plaie ou la peau lésée, et de la positionner aisément sans la toucher.

Dès lors que le pansement est ainsi positionné, il suffit, dans un deuxième temps, de retirer totalement le protecteur en le séparant du pansement au niveau de la face opposée à celle qui est au contact de la plaie ou de la peau lésée.

Selon un deuxième mode de réalisation, l'ensemble pansement-protecteur selon l'invention comprend un pansement interface complexé à une compresse.

De tels pansements sont connus et commercialisés par exemple sous les dénominations commerciales UrgotulDuo^{®} et Cellosorb^{®} (par les Laboratoires Urgo).

Lorsqu'un pansement interface comprenant au moins un corps gras est complexé à une compresse, ou lorsqu'une compresse est enduite d'une masse comprenant au moins un corps gras sur la surface destinée à venir en contact avec la plaie ou la peau lésée, il se produit un phénomène de migration du corps gras à travers la compresse. Ce phénomène peut provoquer une altération de la pochette de conditionnement.

Dans ce type de pansement, la surface de la compresse enduite d'une masse comprenant au moins un corps gras peut facilement être couverte d'un protecteur classique sous forme d'une feuille car celui-ci peut être maintenu sur cette surface grâce aux propriétés d'adhérence de la masse. En revanche, ceci n'est pas le cas pour la surface opposée de la compresse qui ne présente pas de propriété d'adhérence.

L'ensemble pansement-protecteur selon la présente invention permet de résoudre ce problème. En effet, comme on le comprend, du fait que le protecteur est constitué d'une feuille unique, la force d'adhérence fournie au niveau des ailes rabattues sur la face du pansement destinée à venir au contact de la plaie ou de la peau est suffisante pour maintenir le protecteur contre le pansement au niveau de la face opposée de la compresse.

Il a par ailleurs été constaté un phénomène surprenant lors du conditionnement d'un pansement interface complexé à une compresse à l'aide d'un ensemble pansement-protecteur conforme à l'invention. En effet, en fonction des matériaux utilisés, notamment lorsque le protecteur est en polyester non siliconé et la compresse en viscose on a observé un phénomène d'électricité statique entre le protecteur et la compresse, qui permet, contre toute attente, de maintenir la compresse liée au protecteur jusqu'à sa pose sur la plaie ou la peau lésée. Dans ce cas, le protecteur peut également jouer le rôle d'applicateur.

Selon un troisième mode de réalisation, l'ensemble pansement-protecteur selon l'invention comprend un pansement à base d'hydrogels ou d'hydrocolloïdes.

De tels pansements sont connus et commercialisés par exemple sous les dénominations commerciales Algoplaque^{®} (par les Laboratoires Urgo), Duoderm^{®} (par Convatec), Comfeel^{®} (par Coloplast).

Ces derniers sont constitués d'une masse comprenant au moins un corps gras et d'hydrocolloïdes, ladite masse étant complexée à un support adhésivé.

Dans ce type de pansement, l'ensemble pansement-protecteur selon l'invention permet d'éviter les problèmes de fluage de masse au niveau des bords latéraux. Il peut également faciliter l'application du pansement lors de la pose.

D'autres détails, caractéristiques et avantages de l'invention apparaîtront plus clairement à la lecture de la description explicative qui va suivre de plusieurs modes de réalisation de l'invention et en référence aux figures annexées, dans lesquelles :
- la figure 1 représente une vue en perspective d'un pansement interface entouré d'un protecteur selon un premier mode de réalisation de la présente invention ;
- la figure 2 représente une vue en perspective semblable à la figure 1 d'un pansement interface complexé à une compresse mince, entouré d'un protecteur selon un second mode de réalisation de la présente invention ;
- la figure 3 représente une vue en perspective semblable à la figure 1 d'un pansement interface complexé à une compresse épaisse, ladite compresse étant fixée à un support, entouré d'un protecteur selon un troisième mode de réalisation de la présente invention ;
- la figure 4 représente une vue en perspective d'un pansement entouré d'un protecteur selon un quatrième mode de réalisation de l'invention ;
- la figure 5 et une vue en perspective qui représente schématiquement une installation permettant la fabrication de l'ensemble pansement-protecteur selon l'invention.

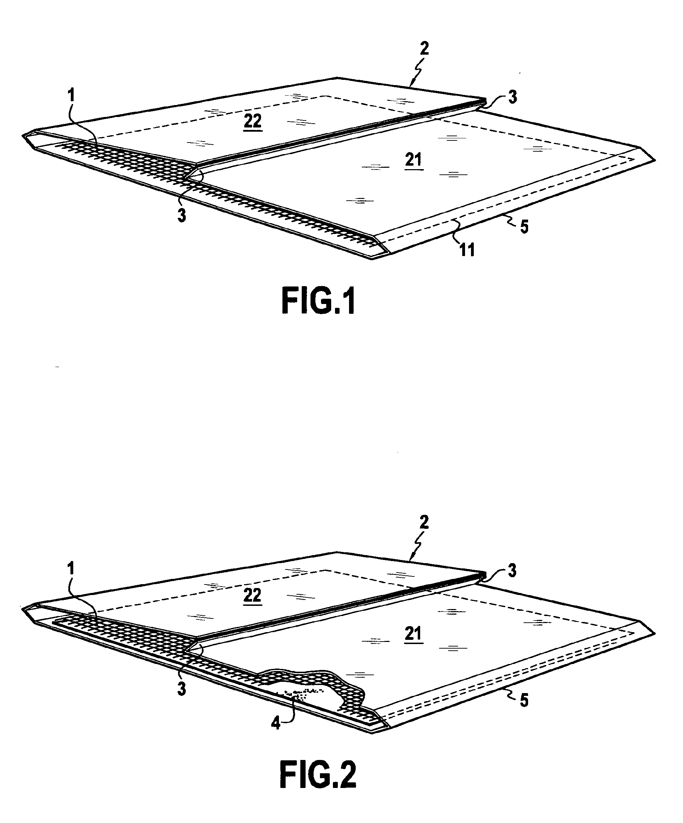

La figure 1 illustre un premier mode de réalisation de l'invention dans lequel un protecteur 2 entoure complètement un pansement interface 1 constitué d'une trame enrobée d'une masse comprenant au moins un corps gras.

Le protecteur 2 est constitué d'une feuille unique qui comprend:
- une première partie (centrale dans l'exemple représenté) recouvrant complètement la face (inférieure dans l'exemple représenté) du pansement 1 opposée à la face destinée à venir au contact de la peau ou de la plaie du patient à traiter et
- deux ailes 21, 22 s'étendant respectivement de part et d'autre de ladite première partie et rabattues sur la face du pansement 1 destinée à venir au contact de la peau ou de la plaie.

Dans l'exemple représenté, les bords libres (c'est-à-dire les bords latéraux) des deux ailes rabattues se recouvrent partiellement en formant ainsi une fente facilitant leur préhension. La surface de recouvrement ainsi définie représente généralement 10 à 30 %, de préférence 10 à 20 %, et de préférence encore 12 à 18 %, de la surface de la face du pansement recouverte par lesdites ailes.

Dans une variante de réalisation non représentée, les bords libres des deux ailes peuvent conformés pour venir en aboutement, en position d'utilisation. Dans ce cas, les ailes précitées pourront s'étendre latéralement au-delà des bords latéraux du pansement pour former des organes de préhension.

Dans l'exemple représenté, la feuille constituant le protecteur 2 est repliée sur elle-même le long d'une amorce de pliage 5 en forme de ligne, qui est disposée en position d'utilisation au niveau (c'est-à-dire au voisinage immédiat ou à quelques millimètres) de l'un des bords latéraux du pansement.

Avantageusement, la feuille peut être également repliée sur elle-même le long d'une seconde ligne de pliage disposée au niveau du bord latéral opposé du pansement.

Pour faciliter le retrait du protecteur, la feuille précitée est repliée sur elle-même au niveau de l'un de ses bords libres en formant ainsi une ailette de préhension 3. Dans l'exemple représenté, la feuille est repliée sur elle-même vers l'extérieur le long d'une ligne droite, mais tout autre pliage connu peut être utilisé pour former l'ailette de préhension.

Dans une variante de réalisation, la feuille peut être repliée sur elle-même au niveau de chacun de ses bords libres en formant ainsi deux ailettes de préhension.

Le pansement interface 1 entouré du protecteur 2 est habituellement conservé dans une pochette de conditionnement.

Dans une variante de réalisation, il peut être envisagé d'écraser le pansement au niveau de ces bords latéraux lors de la formation de la ligne de pliage. Il est ainsi possible d'obtenir des pansements présentant des bords amincis.

Dans ce premier mode de réalisation de l'invention, le protecteur 2 empêche la migration du corps gras contenu dans le pansement vers la pochette de conditionnement, évitant ainsi de la détériorer. Il facilite en outre la pose du pansement en jouant un rôle d'applicateur.

L'épaisseur de la feuille formant le protecteur 2 peut varier de 20 à 200 µm, de préférence de 40 à 80 µm. De manière préférentielle, la feuille utilisée pour former le protecteur selon la présente invention est une feuille de polyester non siliconé ayant une épaisseur de 50 µm.

La figure 2 illustre un deuxième mode de réalisation de l'invention dans lequel le pansement est constitué d'une compresse mince 4 dont l'une des faces est enduite au moins en partie d'une masse comprenant un corps gras ou est complexée à un pansement interface 1 tel que représenté à la figure 1.

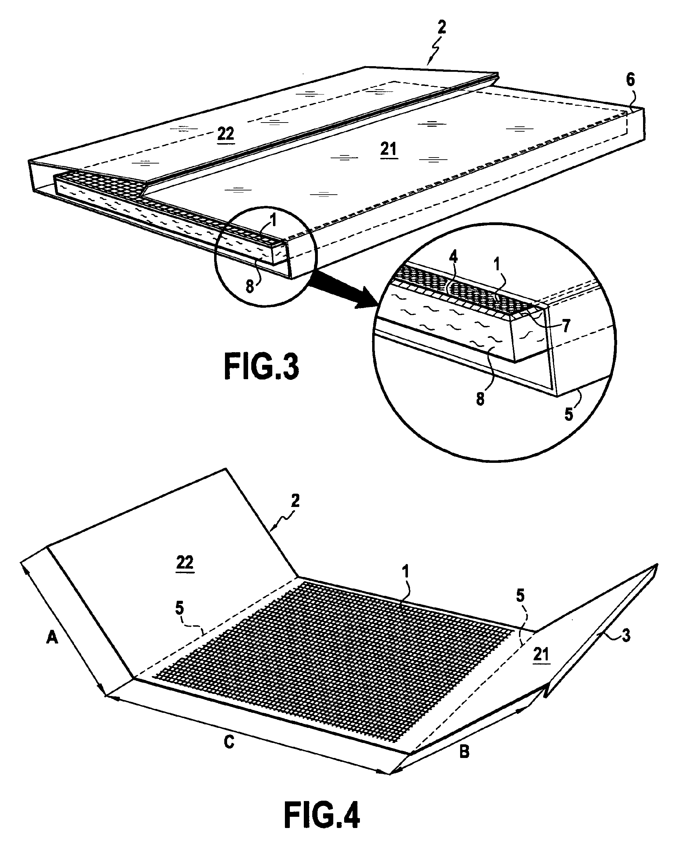

Comme dans le cas du premier mode de réalisation, la feuille constituant le protecteur 2 comporte une ligne de pliage disposée au niveau de chaque bord latéral du pansement 1 bien que l'épaisseur de ce pansement soit supérieure à celle du pansement représenté à la figure 1. D'une façon générale, la feuille peut être repliée facilement autour du pansement lorsque celui-ci présente une épaisseur comprise entre 0,1 et 2 mm. Pour des épaisseurs supérieures, il peut être nécessaire de prévoir deux lignes de pliage pour chacune des ailes du protecteur (voir exemple représenté à la figure 3).

Dans cet exemple, chaque pansement a été découpé à une dimension 10*10 cm et entouré par un protecteur constitué d'une feuille de polyester non siliconé de 50 µm avant d'être conditionné dans une pochette.

Un phénomène d'électricité statique a été observé avec une compresse non tissée 100% viscose dont le poids est de 100 g/m² et l' épaisseur de 0,75 mm (commercialisée sous la dénomination ORSA par JETTEX) complexée à un pansement interface constitué d'une trame en polyester enrobé d'une masse comprenant au moins un corps gras.

Ce phénomène a persisté après conditionnement en pochette.

Ce phénomène peut être amplifié en chargeant le matériau formant le protecteur et/ou la surface de la compresse ou du support qui entre en contact avec le protecteur, à l'aide de barres ionisantes (comme par exemple les barres de charge électrostatique commercialisées par AMG-static ou les systèmes de charge électrostatique commercialisés par HAUG gmbh, ou encore les barres génératrices de charges statiques 991 et 993 commercialisées par Meech Static Eliminators Ltd).

Des essais de routine permettront à l'homme du métier d'observer ou de mettre en évidence ce phénomène avec d'autres matériaux, épaisseurs ou poids du pansement. Il est évident qu'un pansement de poids trop élevé, ou dont la surface de contact avec le protecteur est trop réduite ne pourra être maintenu par un tel lien physique.

Dans ce mode de réalisation, le protecteur peut également jouer le rôle d'applicateur.

La figure 3 représente un troisième mode de réalisation de l'invention dans lequel le pansement est constitué d'un support 8 enduit d'un adhésif 7 permettant de lier le support à une compresse épaisse 4, elle-même complexée à un pansement interface 1 comprenant au moins un corps gras.

Ce pansement présente une épaisseur telle qu'il est nécessaire de former une seconde ligne de pliage 6 au niveau des bords latéraux du protecteur 2 afin que la surface de contact avec les surfaces du pansement soit maximum.

D'une façon générale, il est préférable de prévoir deux lignes de pliage au niveau des bords latéraux du protecteur pour des pansements présentant une épaisseur supérieure à 2 mm.

En fonction de la nature du pansement ou du rôle joué par le protecteur, la feuille formant le protecteur peut être au moins en partie couverte d'un matériau anti-adhérent. Il peut être envisagé, par exemple, que seule la partie du protecteur en contact avec la face du pansement entrant en contact avec la plaie ou la peau lésée soit couverte d'un matériau anti-adhérent.

De même, la feuille formant le protecteur peut être couverte au moins en partie d'un adhésif.

Un tel mode de réalisation est illustré à la figure 4.

Dans cet exemple, le protecteur 2 est recouvert d'un adhésif micro-adhérent au niveau d'une zone (désignée par la lettre C sur la figure) afin d'améliorer son rôle d'applicateur dans certains modes de réalisation. En effet, un adhésif micro-adhérent permettra de maintenir le pansement sur le protecteur jusqu'au moment de sa pose sur la plaie ou la peau lésée.

Dans une variante particulière de réalisation, la zone adhésivée C du protecteur peut servir de support pour le pansement. Dans ce cas, l'adhésif choisi devra avoir des propriétés adhésives suffisantes pour solidariser la masse comprenant au moins un corps gras, complexée ou non à une compresse, au protecteur. En outre, comme le montre la figure 4, le protecteur présentera une amorce de découpe (ou pré-découpe) au niveau de la ligne de pliage 5 permettant le retrait par découpe des parties formant ailes (désignées par les lettres A et B sur la figure) du protecteur en laissant subsister la zone adhésivée C du protecteur sur le pansement.

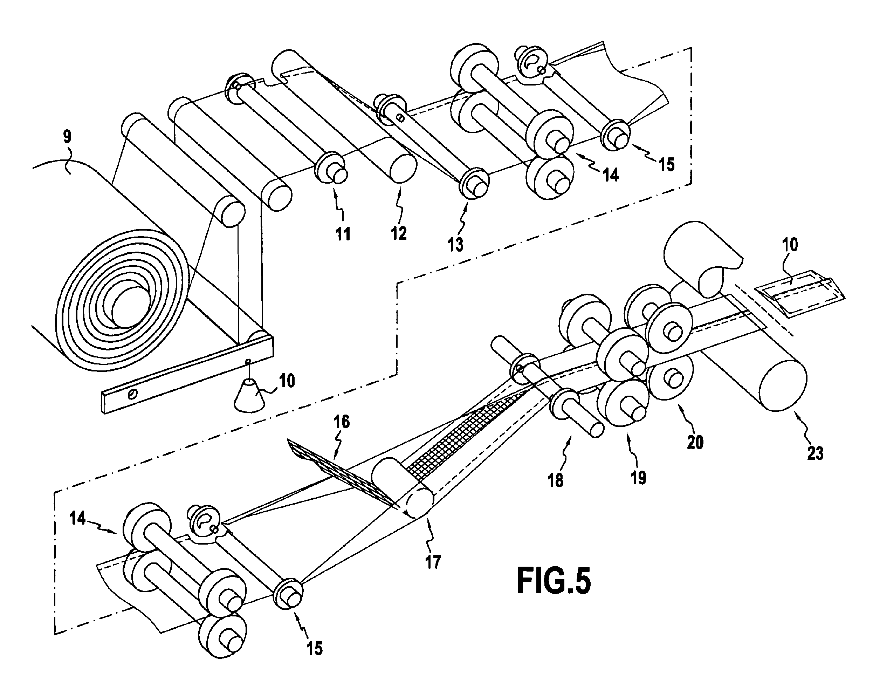

En référence à la figure 5 on décrira maintenant un procédé de fabrication de l'ensemble pansement-protecteur selon la présente invention.

D'une façon générale, ce procédé de fabrication comporte les étapes principales suivantes :
a) arrivée à plat de la feuille formant le protecteur
b) arrivée à plat du pansement à entourer
c) déviation de la feuille formant le protecteur au niveau d'une roue
d) formation de la ligne de pliage au niveau des bords latéraux.

La feuille constituant le protecteur provient d'une bobine amenée à plat à la sortie d'un cylindre 9.

La feuille constituant le protecteur est mise sous tension à l'aide d'un poids 10.

Dans une seconde étape, qui est optionnelle, lorsque le protecteur présente des ailettes de préhension, on vient former un pli au niveau d'un bord latéral de la feuille formant le protecteur. En fonction du type d'ailettes de préhension voulu, un pli peut éventuellement être formé au niveau de chacun des deux bords latéraux de la feuille formant le protecteur. Ce pli est d'abord formé au niveau du poste 12 situé entre deux éléments de guidage fixes 11 et 13, puis il est renforcé par simple pincement mécanique à l'aide d'un galet métallique, en acier par exemple, au niveau du poste 14 situé entre deux éléments de guidage fixes 13 et 15. En fonction du matériau formant le protecteur, il peut être nécessaire d'apporter de la chaleur au système pour faciliter la formation du pli.

Dans une troisième étape, la feuille plane formant le protecteur est déviée à l'aide d'une roue 17, située entre deux éléments de guidage 15 et 18, en dessous du niveau de défilement. De manière préférentielle, on choisira un cylindre de petite dimension afin de réduire la déviation et de diminuer la distance entre le cylindre d'apport de la feuille formant le protecteur 9 (ou 12, si le procédé utilisé comporte la seconde étape optionnelle de formation d'une ailette de préhension) et le cylindre de finition de la ligne de pliage 18.

La roue 17 peut avoir différentes formes : il peut s'agir d'une roue aspirante, notamment pour les cas où la feuille formant le protecteur présente une certaine fragilité. Elle peut être chauffée ou refroidie en fonction des besoins.

L'arrivée du pansement 16 peut se faire en amont de cette roue ou au niveau de cette dernière.

Dans un mode de réalisation de l'invention, il peut être envisagé d'amener la masse comprenant au moins un corps gras au niveau de la paroi de la roue 17 puis celle-ci vient se complexer directement soit à la feuille formant le protecteur soit à une compresse ou un support dont l'arrivée se fait au niveau de la roue ou en amont de celle-ci.

La roue 17 peut également avoir des motifs ou reliefs au niveau de sa surface extérieure, aboutissant ainsi à différentes formes de pansements selon les principes connus de l'héliogravure.

Cette roue peut également présenter des lames ou être couplée à un cylindre présentant des lames permettant de couper le pansement. Ceci peut être avantageux lorsque l'on désire avoir un pansement de dimension inférieure à celle du protecteur.

Suite au passage du protecteur et du pansement contenant une masse comprenant au moins un corps gras au niveau de la roue 17, il se produit un rabattement naturel des zones A et B de la feuille formant le protecteur. Les lignes de pliages au niveau des bords latéraux sont alors renforcées par écrasement par simple pincement mécanique à l'aide de galets métalliques, de préférence en acier au niveau d'un cylindre 19. En fonction du matériau formant le protecteur, il peut être nécessaire d'apporter de la chaleur au système pour faciliter la formation de la ligne de pliage.

Les lignes de pliages peuvent être réalisées au niveau du bord immédiat du pansement ou à plusieurs millimètres de celui-ci. Dans certains modes de réalisation de l'invention, il peut être envisagé d'écraser le pansement au niveau des bords latéraux en même temps que l'on écrase la feuille formant le protecteur afin de former les lignes de pliage. Ceci permet d'obtenir des pansements présentant des bords amincis.

La tension entre le cylindre d'apport de la feuille formant le protecteur 9 et le cylindre de tirage avec coupleur 20 varie en fonction du matériau utilisé pour la feuille formant le protecteur. Lorsque cette feuille est en polyester de 50 µm d'épaisseur, la tension est de préférence située entre 3 et 5 N/cm. La vitesse de déroulement dans ce cas là est de préférence située entre 3 à 50 m/min, l'objectif principal étant de maintenir une tension constante entre la roue 17 et le cylindre 19 pour éviter que la feuille formant le protecteur ne reprenne une conformation plane.

On procède ensuite à une découpe du protecteur ou du protecteur et du pansement selon le procédé de fabrication utilisé au niveau d'un cylindre 23 afin d'obtenir un pansement individuel 10 entouré complètement d'un protecteur.

Dans une variante de réalisation de l'invention, il peut être envisagé, lorsque la dimension du pansement est inférieure à celle du protecteur, de réaliser une soudure au niveau des deux extrémités du protecteur afin de protéger le pansement de toute contamination extérieure ou d'éviter que le corps gras ne migre à ce niveau là.

## Revendications

1. Ensemble pansement-protecteur, du type comprenant :
- un pansement incorporant au moins un corps gras et présentant une première face destinée à venir au contact de la peau ou de la plaie et une deuxième face opposée à la première ;
- un protecteur séparable dudit pansement,
**caractérisé en ce que** ledit protecteur est constitué d'une feuille unique recouvrant complètement ladite deuxième face et présentant des ailes rabattues sur ladite première face, de façon à la recouvrir sensiblement entièrement.

2. Ensemble selon la revendication 1, **caractérisé en ce que** les ailes rabattues précitées se recouvrent partiellement au niveau de leurs bords libres, de préférence de telle façon que la surface de recouvrement ainsi définie représente 10 à 30 %, de préférence encore 10 à 20 % de la surface de la face du pansement recouverte par lesdites ailes.

3. Ensemble selon la revendication 1 ou 2, **caractérisé en ce que** la feuille précitée constituant le protecteur comprend au moins une amorce de pliage destinée à être positionnée au niveau d'un bord latéral du pansement et permettant son repli sur elle-même pour former l'une des ailes précitées.

4. Ensemble selon l'une des revendications 1 à 3, **caractérisé en ce que** la feuille précitée constituant le protecteur comprend au moins deux amorces de pliage destinées à être positionnées respectivement au niveau de chacun des bords latéraux du pansement et permettant son repli sur elle-même pour former respectivement les deux ailes précitées.

5. Ensemble selon l'une des revendications 1 à 4, **caractérisé en ce que** la feuille précitée constituant le protecteur est repliée sur elle-même au niveau de l'un de ses bords libres en formant ainsi une ailette de préhension.

6. Ensemble selon l'une des revendications 1 à 4, **caractérisé en ce que** la feuille précitée constituant le protecteur est repliée sur elle-même au niveau de chacun de ses bords libres en formant ainsi deux ailettes de préhension.

7. Ensemble selon l'une des revendications 1 à 6, **caractérisé en ce que** la feuille précitée constituant le protecteur est formée d'un matériau au travers duquel le corps gras ne migre pas, ledit matériau étant de préférence choisi parmi un papier siliconé, le polyéthylène ou un polyester.

8. Ensemble selon l'une des revendications 1 à 7, **caractérisé en ce que** la feuille précitée constituant le protecteur présente une épaisseur comprise entre 20 et 200 µm, de préférence entre 40 et 80 µm.

9. Ensemble selon l'une des revendications 1 à 8, **caractérisé en ce que** la feuille précitée constituant le protecteur est recouverte au moins en partie sur une de ses faces par un matériau anti-adhérent.

10. Ensemble selon l'une des revendications 1 à 9, **caractérisé en ce que** la feuille précitée constituant le protecteur est recouverte au moins en partie sur une de ses faces, d'un matériau adhésif.

11. Ensemble selon l'une des revendications 1 à 10, **caractérisé en ce que** la feuille précitée constituant le protecteur présente, au niveau de chacune des amorces de pliage, des moyens de pré-découpe.

12. Procédé de fabrication d'un ensemble pansement-protecteur selon l'une des revendications 1 à 11 comprenant les étapes suivantes :
a) arrivée à plat de la feuille formant le protecteur
b) arrivée à plat du pansement à entourer
c) déviation de la feuille formant le protecteur au niveau d'une roue 17
d) formation de la ligne de pliage au niveau des bords latéraux

13. Procédé de fabrication selon la revendication 12 comprenant une étape supplémentaire entre les étapes a) et b) dans laquelle un pli est formé au niveau d'au moins un des bords libres de la feuille afin de former une ailette de préhension.

14. Procédé de fabrication selon l'une des revendications 12 ou 13, **caractérisé en ce que** la roue 17 est chauffée, refroidie, aspirante, non-aspirante, lisse, présente des motifs ou reliefs au niveau de sa surface extérieure.

15. Procédé de fabrication selon l'une des revendications 12 à 14, **caractérisé en ce que** la masse comprenant au moins un corps gras est amenée au niveau de la paroi de la roue 17.

## Claims

1. A dressing-protector system comprising:
• a dressing incorporating at least one fatty substance and having a first face intended to come into contact with skin or a wound and a second face that is opposite the first;
• a protector that can be separated from said dressing;
the system being **characterized in that** said protector is constituted by a single sheet that completely covers said second face, having flaps folded down over said first face in order to cover it substantially completely.

2. A system according to claim 1, **characterized in that** said folded down flaps partially overlap at their free edges, preferably such that the overlap surface area defined thereby represents 10% to 30%, more preferably 10% to 20% of the surface area of the face of the dressing covered by said flaps.

3. A system according to claim 1 or claim 2, **characterized in that** said sheet constituting the protector includes at least one fold starter intended to be positioned at a lateral edge of the dressing and allowing it to be folded onto itself to form one of said flaps.

4. A system according to any one of claims 1 to 3, **characterized in that** said sheet constituting the protector includes at least two fold starters intended to be positioned respectively at each of the lateral edges of the dressing so that it enables said sheet to be folded onto itself thereby forming the two said flaps respectively.

5. A system according to any one of claims 1 to 4, **characterized in that** said sheet constituting the protector is folded onto itself at one of its free edges, thereby forming a grasping margin.

6. A system according to any one of claims 1 to 4, **characterized in that** said sheet constituting the protector is folded onto itself at each of its free edges, thereby forming two grasping margins.

7. A system according to any one of claims 1 to 6, **characterized in that** said sheet constituting the protector is formed from a material through which the fatty substance does not migrate, said material preferably being selected from a silicone paper, polyethylene, or a polyester.

8. A system according to any one of claims 1 to 7, **characterized in that** the thickness of said sheet constituting the protector is in the range of 20 µm to 200 µm, preferably in the range 40 µm to 80 µm.

9. A system according to any one of claims 1 to 8, **characterized in that** at least a portion of one of the faces of said sheet constituting the protector is covered with a non-adhering material.

10. A system according to any one of claims 1 to 9, **characterized in that** at least a portion of one of the faces of said sheet constituting the protector is covered with an adhesive material.

11. A system according to any one of claims 1 to 10, **characterized in that** said sheet constituting the protector has pre-cut means at each of the fold starters.

12. A method of manufacturing a dressing-protector system according to any one of claims 1 to 11, comprising the following steps:
a) flat feeding a sheet forming the protector;
b) flat feeding a dressing to be surrounded;
c) deflecting the sheet forming the protector at a wheel (17);
d) forming a fold line at the lateral edges.

13. A method of manufacturing according to claim 12, comprising an additional step between steps a) and b) in which a fold is formed at at least one of the free edges of the sheet in order to form a grasping margin.

14. A method of manufacturing according to claim 12 or claim 13, **characterized in that** the wheel (17) is heated, cooled, is a suction or non-suction wheel, is smooth, or has patterns or embossments on its external surface.

15. A method of manufacturing according to any one of claims 12 to 14, **characterized in that** the mass comprising at least one fatty substance is fed to the wall of the wheel (17).

## Patentansprüche

1. Verband-Schutz-Anordnung, vom Typ umfassend:
- einen Verband, der wenigstens ein Fett einschließt und eine erste Seite, die dazu bestimmt ist, mit der Haut oder der Wunde in Kontakt zu kommen, sowie eine der ersten gegenüberliegende zweite Seite aufweist,
- einen von dem Verband ablösbaren Schutz,
**dadurch gekennzeichnet, daß** der Schutz von einer einzigen Folie gebildet ist, welche die zweite Seite vollständig bedeckt und Flügel aufweist, die auf die erste Seite heruntergeklappt sind, um sie im wesentlichen vollständig zu bedecken.

2. Anordnung nach Anspruch 1, **dadurch gekennzeichnet, daß** die vorgenannten heruntergeklappten Flügel sich im Bereich ihrer freien Kanten teilweise überlappen, vorzugsweise derart, daß die so definierte Überlappungsfläche 10 bis 30 %, weiterhin vorzugsweise 10 bis 20 % der Fläche der durch die Flügel bedeckten Seite des Verbands ausmacht.

3. Anordnung nach Anspruch 1 oder 2, **dadurch gekennzeichnet, daß** die den Schutz bildende vorgenannte Folie wenigstens eine Faltansatzstelle aufweist, die dazu bestimmt ist, im Bereich einer Seitenkante des Verbands positioniert zu werden, und die ermöglicht, sie auf sich selbst umzuschlagen, um einen der vorgenannten Flügel zu bilden.

4. Anordnung nach einem der Ansprüche 1 bis 3, **dadurch gekennzeichnet, daß** die den Schutz bildende vorgenannte Folie wenigstens zwei Faltansatzstellen aufweist, die dazu bestimmt sind, jeweils im Bereich einer jeden der Seitenkanten des Verbands positioniert zu werden, und die ermöglichen, sie auf sich selbst umzuschlagen, um jeweils die beiden vorgenannten Flügel zu bilden.

5. Anordnung nach einem der Ansprüche 1 bis 4, **dadurch gekennzeichnet, daß** die den Schutz bildende vorgenannte Folie im Bereich einer ihrer freien Kanten auf sich selbst umgeschlagen ist, wodurch ein Greifflügelchen gebildet wird.

6. Anordnung nach einem der Ansprüche 1 bis 4, **dadurch gekennzeichnet, daß** die den Schutz bildende vorgenannte Folie im Bereich einer jeden ihrer freien Kanten auf sich selbst umgeschlagen ist, wodurch zwei Greifflügelchen gebildet werden.

7. Anordnung nach einem der Ansprüche 1 bis 6, **dadurch gekennzeichnet, daß** die den Schutz bildende vorgenannte Folie aus einem Material gebildet ist, durch das das Fett nicht hindurch wandert, wobei das Material vorzugsweise aus einem silikonbeschichteten Papier, Polyethylen oder einem Polyester ausgewählt ist.

8. Anordnung nach einem der Ansprüche 1 bis 7, **dadurch gekennzeichnet, daß** die den Schutz bildende vorgenannte Folie eine Dicke zwischen 20 und 200 µm, vorzugsweise zwischen 40 und 80 µm aufweist.

9. Anordnung nach einem der Ansprüche 1 bis 8, **dadurch gekennzeichnet, daß** die den Schutz bildende vorgenannte Folie auf einer ihrer Seiten wenigstens teilweise mit einem Antihaftmaterial überzogen ist.

10. Anordnung nach einem der Ansprüche 1 bis 9, **dadurch gekennzeichnet, daß** die den Schutz bildende vorgenannte Folie auf einer ihrer Seiten wenigstens teilweise mit einem Haftmaterial überzogen ist.

11. Anordnung nach einem der Ansprüche 1 bis 10, **dadurch gekennzeichnet, daß** die den Schutz bildende vorgenannte Folie im Bereich einer jeden der Faltansatzstellen Vorschneidemittel aufweist.

12. Verfahren zur Herstellung einer Verband-Schutz-Anordnung nach einem der Ansprüche 1 bis 11, welches die folgenden Schritte umfaßt:
a) Zuführen der den Schutz bildenden Folie in flachem Zustand,
b) Zuführen des zu umschließenden Verbandes in flachem Zustand,
c) Ablenken der den Schutz bildenden Folie im Bereich eines Rades (17),
d) Bilden der Faltlinie im Bereich der Seitenkanten.

13. Herstellungsverfahren nach Anspruch 12, umfassend einen zusätzlichen Schritt zwischen den Schritten a) und b), bei dem im Bereich wenigstens einer der freien Kanten der Folie ein Knick ausgebildet wird, um ein Greifflügelchen zu bilden.

14. Herstellungsverfahren nach einem der Ansprüche 12 oder 13, **dadurch gekennzeichnet, daß** das Rad (17) beheizt, gekühlt, ansaugend, nicht ansaugend, glatt ist, Muster oder Reliefs im Bereich seiner Außenfläche aufweist.

15. Herstellungsverfahren nach einem der Ansprüche 12 bis 14, **dadurch gekennzeichnet, daß** die wenigstens ein Fett enthaltende Masse im Bereich der Wand des Rades (17) zugeführt wird.
